# EUROPÄISCHE PATENTANMELDUNG

(11) **EP 3 884 851 A1**
(43) Veröffentlichungstag der Anmeldung: **29.09.2021**
(21) Anmeldenummer: 21164152.7
(22) Anmeldetag: 23.03.2021
(51) Int. Cl.: A61B 5/00, H02H 3/16

(54) **VERFAHREN UND VORRICHTUNG ZUM SCHUTZ GEGEN ELEKTRISCHEN SCHLAG DURCH ERFASSUNG UND BEWERTUNG PHYSIKALISCH-MEDIZINISCHER GRÖSSEN**

(30) Priorität: 26.03.2020 DE 102020108390
(71) Anmelder: Bender GmbH & Co. KG, 35305 Grünberg (DE)
(72) Erfinder: HACKL, Dieter, 35463 Fernwald (DE)
(74) Vertreter: advotec.

(57) **Zusammenfassung**

Die Erfindung betrifft ein Verfahren und eine Vorrichtung zum Schutz einer Person gegen elektrischen Schlag mit einer Erfassung einer physikalisch-medizinischen Größe mittels einer an dem Körper (4) der Person angeordneten Sensoreinrichtung (6), mit einer Bewertung der als Sensordaten (20) von der Sensoreinrichtung (6) erfassten physikalisch-medizinischen Größe mittels einer Bewertungseinrichtung (8), ob eine Gefährdung durch einen Körperstrom (I_{B}) zu erwarten ist, und mit einer Abschaltung einer der Person zugeordneten, den möglichen Körperstrom (I_{B}) verursachenden Stromversorgung (12) mittels einer Abschalteinrichtung (10), falls die Gefährdung besteht.

## Beschreibung

Die Erfindung betrifft ein Verfahren und eine Vorrichtung zum Schutz einer Person gegen elektrischen Schlag.

Statistische Untersuchungen für Deutschland zeigen, dass zwar im Laufe der zurückliegenden Jahre aufgrund der immer weiter optimierten Schutz- und Schulungsmaßnahmen beim Umgang mit elektrischem Strom im betrieblichen Umfeld im Mittel ein kontinuierlicher Rückgang der Anzahl von tödlichen Stromunfällen zu verzeichnen ist, aber die Anzahl gemeldeter Stromunfälle zunimmt. Weiterhin ist den Untersuchungen zu entnehmen, dass bei über 90 % dieser gemeldeten Stromunfälle gefährliche Körperströme als Ursache genannt werden.

Die zusammenhängende Betrachtung des Rückgangs von tödlichen Stromunfällen und der Zunahme von gemeldeten Stromunfällen mit einer physiologisch kritischen Körperdurchströmung als überwiegende Ursache für die Stromunfälle legt den Schluss nahe, dass nach dem Stand der Technik geltende Schutzmaßnahmen und Schutzeinrichtungen bestimmte Schutzlücken aufweisen.

Eine maßgebliche Schutzlücke besteht in der Differenz zwischen der Stromempfindlichkeit des Menschen gegenüber Körperdurchströmungen und der Auslöseempfindlichkeit von Fehlerstrom-Schutzeinrichtungen nach dem Stand der Technik.

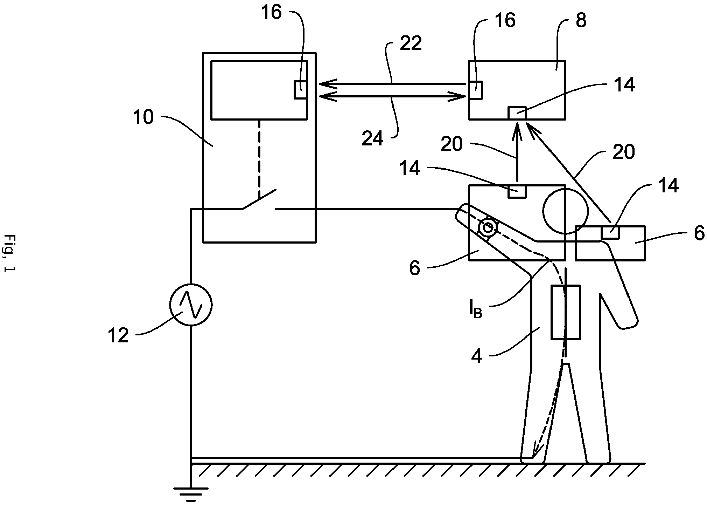

Der internationale Standard IEC60479-1:2018 "Effects of current on human beings and livestock - Part 1: General aspects" benennt in einem Zeit-Stromstärke-Diagramm der physiologischen Wirkungen von Körperströmen gemäß Fig. 20 eine Grenzkurve b für Körperwechselströme im Frequenzbereich von 15Hz bis 100Hz, welche zwischen der linken Hand und den Füßen eines Menschen fließen, und bei deren Überschreitung mit beeinträchtigenden physiologischen Wirkungen zu rechnen ist. Diese Auswirkungen treten oberhalb der Grenzlinie b bis zu einer Grenzlinie c₁ in einem Bereich AC-3 auf und äußern sich beispielsweise in Form von starken unwillkürlichen Muskelkontraktionen, Schwierigkeiten beim Atmen oder reversible Störungen der Herzfunktion.

Die Grenzlinie b wird ab einem Körperstrom von 5mA und einer Durchströmungsdauer von ca. 7s überschritten. Oberhalb dieser als Loslass-Schwelle bezeichneten Grenzlinie b von 5mA kann der Mensch sich bei einsetzender Muskelverkrampfung nicht mehr zuverlässig aus der Körperstromsituation befreien.

Demgegenüber müssen laut Produktnorm IEC61008-1 Fehlerstrom-Schutzeinrichtungen (RCD) erst ab einem Nennfehlerstrom von 30mA sicher auslösen und dürfen, um Fehlauslösungen zu vermeiden, nicht bei einem Fehlerstrom unterhalb von 15mA auslösen.

Es bleibt somit eine Schutzlücke zwischen der Loslass-Schwelle von 5mA für Körperdurchstömungen und dem Auslösefehlerstrom von 15mA, bzw. im ungünstigsten Fall sogar von 30mA, bei Fehlerstrom-Schutzeinrichtungen bestehen.

Um diesem Problem entgegenzutreten wurden bislang neben dem Einsatz der bereits genannten Fehlerstrom-Schutzeinrichtungen der Einsatz von Schutzausrüstung (Arbeitsschuhe, Handschuhe, schutzisoliertes Werkzeug) anwendungsspezifisch vorgegeben und man ist bestrebt, durch nicht-technische Maßnahmen die Unfallzahl durch Information, Aufklärung, Schulung und durch die Vorgabe von Sicherheitsregeln zu reduzieren. Allerdings steht diesen Bemühungen für mehr Sicherheit und weniger Unfälle eine zunehmende zeit- und kosteneffiziente Arbeitsweise entgegen, sodass die bisherigen Schutzmaßnahmen oft umgangen und Sicherheitsregeln missachtet werden.

Der vorliegenden Erfindung liegt somit die Aufgabe zugrunde ein Verfahren und eine Vorrichtung zum Schutz gegen elektrischen Schlag vorzuschlagen, welche in der Lage sind, mögliche gefährliche Körperströme vorrausschauend zu erkennen und dabei insbesondere die Schutzlücke für Körperströme zwischen 5mA und 15mA bzw. 30mA berücksichtigen.

Diese Aufgabe wird durch ein Verfahren zum Schutz einer Person gegen elektrischen Schlag gelöst, welche die folgenden Verfahrensschritte umfasst:
- Erfassen einer physikalisch-medizinischen Größe mittels einer an dem Körper der Person angeordneten Sensoreinrichtung,
- Bewerten der als Sensordaten von der Sensoreinrichtung erfassten physikalisch-medizinischen Größe mittels einer Bewertungseinrichtung, ob eine Gefährdung durch einen Körperstrom zu erwarten ist,
- Abschalten einer der Person zugeordneten, den möglichen Körperstrom verursachenden Stromversorgung mittels einer Abschalteinrichtung, falls die Gefährdung besteht.

In vorteilhafterweise werden weiter eine oder mehrere der folgenden physikalisch-medizinischen Größen erfasst: Beschleunigung, Herzfrequenz, Blutdruck, Körpertemperatur.

Der Grundgedanke der vorliegenden Erfindung beruht somit darauf, über eine festgelegte Zeitdauer, in der Regel während eines Arbeitseinsatzes, physikalisch-medizinische Größen wie beispielsweise die Beschleunigung des menschlichen Körpers oder eines Körperteils, die Herzfrequenz, den Blutdruck oder die Körpertemperatur an dem Körper der Person zu erfassen, diese Sensordaten dahingehend zu bewerten, ob eine Gefährdung durch einen zu hohen Körperstrom zu erwarten ist und, falls diese Gefährdung besteht, die Stromversorgung, die den möglichen Körperstrom verursachen würde, die also der agierenden Person zugeordnet ist, abzuschalten.

Um gefährliche Körperströme vorausschauend zu erkennen und damit Gefahren abzuwenden, findet somit eine erfindungsgemäße Kopplung zwischen Erfassung und Bewertung körperbezogener Sensordaten einerseits - beruhend auf physikalisch-medizinischen Größen - und der Abschaltung der Stromversorgung andererseits statt.

Das funktionelle Zusammenwirken ausgehend von dem Erfassen einer physikalisch-medizinischen Größe, also dem Messen von Körperfunktionen, mit dem Bewerten der Sensordaten und dem Abschalten der Stromversorgung ermöglichen es, einen für das Individuum physiologisch kritischen Zustand zu erkennen und daraufhin eine selektive automatische Abschaltung der Stromversorgung zu bewirken.

In weiterer vorteilhafter Ausgestaltung werden die Sensordaten der von verschiedenen Sensoreinrichtungen erfassten physikalisch-medizinischen Größen zusammengeführt.

Mit dem Ziel einen physiologisch kritischen Zustand frühzeitig und zuverlässig zu erkennen, werden die Sensordaten verschiedener Sensoreinrichtungen zusammengeführt und gemeinsam, einander unterstützend, ausgewertet.

Bevorzugt erfolgt das Bewerten der Sensordaten zur vorausschauenden Erkennung eines möglichen, oberhalb einer normativ festgelegten Loslass-Schwelle liegenden Körperstroms.

Die Bewertung der Sensordaten zielt also bevorzugt auf die vorausschauende Erkennung eines möglichen Körperstroms ab, der die normativ festgelegte Loslass-Schwelle von 5mA überschreiten könnte.

Mit Vorteil erfolgt das Bewerten mittels digitaler Signalverarbeitungsalgorithmen.

Um aus den erfassten und zusammengeführten Sensordaten vorausschauend ein Bild über mögliche physiologisch kritische Zustände abzuleiten, werden digitale Signalverarbeitungsalgorithmen eingesetzt. Die Verarbeitungsvorschriften können beispielsweise Algorithmen zur Filterung, Glättung oder Prädiktion sein wie sie beispielsweise aus der medizinischen Diagnostik zur Erkennung von Artefakten bekannt sind. Auch können die Bewertungen auf statistischen Signalbeschreibungen beruhen, um Fehler in realen Messwerten zu reduzieren und Schätzungen für nichtmessbare Größen zu liefern. Schließlich sind auch Algorithmen aus dem Bereich der künstlichen Intelligenz möglich.

Weiterhin erfolgt die Übertragung der Sensordaten drahtgebunden oder drahtlos mittels einer Sensordaten-Übertragungseinrichtung.

Falls die Sensoreinrichtung und die Bewertungseinrichtung nicht in einer integrierten baulichen Einheit zusammengefasst sind, kann die Übertragung der Sensordaten zwischen der Sensoreinrichtung und der Bewertungseinrichtung drahtgebunden über eine Kabelverbindungsstrecke oder drahtlos über einer Funkübertragungsstrecke erfolgen. Eine Sensordaten-Übertragungseinrichtung umfasst dazu jeweils einen in der Sensoreinrichtung und der Bewertungseinrichtung angeordneten Daten-Sende-/Empfangsteil.

Mit Vorteil erfolgt die Zuordnung und Abschaltung der Stromversorgung drahtgebunden oder drahtlos mittels einer Abschaltsignal-Übertragungseinrichtung.

Um die selektive automatische Abschaltung einer den möglichen Körperstrom verursachenden Stromversorgung zu bewirken, kann die Zuordnung der abzuschaltenden Stromversorgung zu der gefährdeten Person und die Übertragung eines Abschaltsignale mittels der Abschaltsignal-Übertragungseinrichtung drahtgebunden oder drahtlos erfolgen.

Dabei überträgt eine jeweils in der Bewertungseinrichtung und der Abschalteinrichtung angeordnete Signal-Sende-/Empfangseinheit der Abschaltsignal-Übertragungseinrichtung zunächst Identifikationsdaten, um die der Person zugeordnete Stromversorgung zu identifizieren. Anschließend erfolgt die Übertragung eines Abschaltsignals von der Bewertungseinrichtung zu der Abschalteinrichtung, die daraufhin die den möglichen Körperstrom verursachende Stromversorgung trennt.

Im Fall einer drahtlosen Übertragung bieten sich standardisierte Verfahren auf Basis der RFID-, Bluetooth- oder WLAN-Technologie an.

Die der Erfindung zugrundeliegende Aufgabe wird weiterhin gelöst durch eine Vorrichtung zum Schutz einer Person gegen elektrischen Schlag, mit einer an dem Körper der Person angeordneten Sensoreinrichtung zum Erfassen einer physikalisch-medizinischen Größe, einer Bewertungseinrichtung zum Bewerten der als Sensordaten von der Sensoreinrichtung erfassten physikalisch-medizinischen Größe im Hinblick darauf, ob eine Gefährdung durch einen Körperstrom zu erwarten ist, mit einer Abschalteinrichtung zum Abschalten einer der Person zugeordneten, den möglichen Körperstrom verursachenden Stromversorgung, falls die Gefährdung besteht.

In Umsetzung und zur Ausführung des erfindungsgemäßen Verfahrens weist die erfindungsgemäße Vorrichtung eine Sensoreinrichtung, eine Bewertungseinrichtung und eine Abschalteinrichtung auf.

Insoweit treffen auch die vorgenannten verfahrensbezogenen technischen Wirkungen und die daraus entstehenden Vorteile auf die Vorrichtungsmerkmale zu.

Insbesondere weist die erfindungsgemäße Vorrichtung eine oder mehrere Sensoreinrichtung(en) wie einen Beschleunigungssensor, einen Herzfrequenzsensor, ein Blutdruckmessgerät oder einen Temperatursensor auf.

Die Sensoreinrichtungen können als separate Messinstrumente vorliegen oder beispielsweise in intelligente Kleidung (Smartwear) integriert sein oder mit anderen technischen Einrichtungen eine bauliche Einheit bilden.

Insbesondere können die Sensoreinrichtungen und die Bewertungseinrichtung in einer integrierten baulichen Einheit, beispielsweise in Form eines Smartphones oder eines tragbaren Computersystems, sogenannte Wearables wie Smartwatch, Fitnessarmband oder digitale Brillen, ausgeführt sein.

Die Bewertungseinrichtung ist bevorzugt konfiguriert zur Zusammenführung der Sensordaten der von den verschiedenen Sensoreinrichtungen erfassten physikalisch-medizinischen Größen, zur vorausschauenden Erkennung eines möglichen, oberhalb einer normativ festgelegten Loslass-Schwelle liegenden Körperstroms und zur Bewertung der Sensordaten mittels digitaler Signalverarbeitungsalgorithmen.

Mit Vorteil weist die erfindungsgemäße Vorrichtung eine Sensordaten-Übertragungseinrichtung zur drahtgebundenen oder drahtlosen Übertragung der Sensordaten auf, falls die Sensoreinrichtung und die Bewertungseinrichtung als getrennte bauliche Einheiten ausgestaltet sind.

Weiter umfasst die erfindungsgemäße Vorrichtung eine Abschaltsignal-Übertragungseinrichtung zur Zuordnung und Abschaltung der Stromversorgung.

Die Abschaltsignal-Übertragungseinrichtung überträgt mittels eines drahtgebundenen oder drahtlosen Übertragungsverfahrens Identifikationsdaten und ein Abschaltsignal an die Abschalteinrichtung.

Weitere vorteilhafte Ausgestaltungsmerkmale ergeben sich aus der nachfolgenden Beschreibung und der Zeichnung, die eine bevorzugte Ausführungsform der Erfindung anhand eines Beispiels erläutert.

Es zeigt die
- **Figur:**: die erfindungsgemäße Vorrichtung in funktioneller Darstellung.

Die **Figur** ist eine funktionelle Darstellung der erfindungsgemäßen (Schutz-) Vorrichtung 2 in Verbindung mit einer Person, deren Körper 4 bei Arbeiten an einer zugeordneten Stromversorgung 12 von einem Körperstrom I_{B} durchströmt wird.

Mit einer oder mehreren Sensoreinrichtungen 6 werden physikalisch-medizinische Größen wie beispielsweise Beschleunigung, Herzfrequenz, Blutdruck oder Körpertemperatur aufgenommen und als Sensordaten 20 in elektronisch auswertbarer Form erfasst.

Die Sensoreinrichtungen 6 können als eigenständige Sensoren, beispielsweise Elektroden, oder insbesondere auch als integraler Bestandteil von sogenannten Wearables ausgeführt sein.

Die Sensordaten 20 werden in einer Bewertungseinrichtung 8 zusammengeführt und geschlossen dahingehend ausgewertet, ob eine Gefährdung durch einen möglichen Körperstrom I_{B} zu erwarten ist.

Um einen möglichen, durch den Körperstrom I_{B} ausgelösten, kritischen physiologischen Zustand des menschlichen Körpers 4 vorausschauend zu erkennen, ist die Bewertungseinrichtung 8 zur Ausführung digitaler Signalverarbeitungsalgorithmen ausgelegt und umfasst bevorzugt als Recheneinheit einen Mikroprozessor. Insbesondere sind die Algorithmen zur Auswertung der Sensordaten 20 so programmiert, dass ein Körperstrom erkannt wird, der die normativ festgelegte Loslass-Schwelle von 5mA überschreiten könnte.

Die Sensoreinrichtung 6 und die Bewertungseinrichtung 8 können eine integrale bauliche Einheit bilden und beispielsweise als Wearables von der Person am Körper 4 getragen werden.

Beispielhaft sind in der Figur die Sensoreinrichtung 6 und die Bewertungseinrichtung 8 als getrennte Einrichtungen ausgeführt, sodass die Übertragung der Sensordaten 20 bevorzugt drahtlos mittels einer Sensordaten-Übertragungseinrichtung 14 erfolgt, die jeweils einen in der Sensoreinrichtung 6 und der Bewertungseinrichtung 8 angeordneten Daten-Sende-/Empfangsteil umfasst.

Ergibt die Auswertung der Sensordaten 20 in der Bewertungseinrichtung 8 Anzeichen auf einen physiologisch kritischen Zustand, so leitet die Bewertungseinrichtung 8 ein Abschaltsignal 22 an eine der Person zugeordnete Abschalteinrichtung 10 weiter, um die zugeordnete Stromversorgung 12 abzuschalten.

Die Übertragung des Abschaltsignals 22 erfolgt in diesem Beispiel drahtlos mittels einer Abschaltsignal-Übertragungseinrichtung 16, welche jeweils eine in der Bewertungseinrichtung 8 und der Abschalteinrichtung 10 angeordnete Signal-Sende-/Empfangseinheit umfasst.

Um die Zuordnung zwischen der handelnden Person und der abzuschaltenden Stromversorgung 12 herzustellen, werden zusätzlich Identifizierungsdaten 24 zwischen der Bewertungseinrichtung 8 und der Abschalteinrichtung 10 über die Abschaltsignal-Übertragungseinrichtung 16 übermittelt.

Dazu kann das in der Abschaltsignal-Übertragungseinrichtung 16 verwendete drahtlose Übertragungsverfahren auf bekannten Verfahren wie RFID, Bluetooth oder WLAN beruhen.

## Patentansprüche

1. Verfahren zum Schutz einer Person gegen elektrischen Schlag, umfassend die Verfahrensschritte:
Erfassen einer physikalisch-medizinischen Größe mittels einer an dem Körper (4) der Person angeordneten Sensoreinrichtung (6),
Bewerten der als Sensordaten (20) von der Sensoreinrichtung (6) erfassten physikalisch-medizinischen Größe mittels einer Bewertungseinrichtung (8), ob eine Gefährdung durch einen Körperstrom (I_{B}) zu erwarten ist,
Abschalten einer der Person zugeordneten, den möglichen Körperstrom (I_{B}) verursachenden Stromversorgung (12) mittels einer Abschalteinrichtung (10), falls die Gefährdung besteht.

2. Verfahren nach Anspruch 1,
**dadurch gekennzeichnet,**
**dass** eine oder mehrere der folgenden physikalisch-medizinischen Größen erfasst werden: Beschleunigung, Herzfrequenz, Blutdruck, Körpertemperatur.

3. Verfahren nach Anspruch 1 oder 2,
**gekennzeichnet durch**
Zusammenführen der Sensordaten (20) der von verschiedenen Sensoreinrichtungen (6) erfassten physikalisch-medizinischen Größen.

4. Verfahren nach einem der Ansprüche 1 bis 3,
**dadurch gekennzeichnet,**
**dass** das Bewerten der Sensordaten (20) zur vorausschauenden Erkennung eines möglichen, oberhalb einer normativ festgelegten Loslass-Schwelle liegenden Körperstroms (I_{B}) erfolgt.

5. Verfahren nach einem der Ansprüche 1 bis 4,
**dadurch gekennzeichnet,**
**dass** das Bewerten mittels digitaler Signalverarbeitungsalgorithmen erfolgt.

6. Verfahren nach einem der Ansprüche 1 bis 5,
**dadurch gekennzeichnet,**
**dass** die Übertragung der Sensordaten drahtgebunden oder drahtlos mittels einer Sensordaten-Übertragungseinrichtung (14) erfolgt.

7. Verfahren nach einem der Ansprüche 1 bis 6,
**dadurch gekennzeichnet,**
**dass** die Zuordnung und Abschaltung der Stromversorgung (12) drahtgebunden oder drahtlos mittels einer Abschaltsignal-Übertragungseinrichtung (16) erfolgt.

8. Vorrichtung (2) zum Schutz einer Person gegen elektrischen Schlag, mit
einer an dem Körper (4) der Person angeordneten Sensoreinrichtung zum Erfassen einer physikalisch-medizinischen Größe,
einer Bewertungseinrichtung (8) zum Bewerten der als Sensordaten (20) von der Sensoreinrichtung (6) erfassten physikalisch-medizinischen Größe, ob eine Gefährdung durch einen Körperstrom (I_{B}) zu erwarten ist,
mit einer Abschalteinrichtung (10) zum Abschalten einer der Person zugeordneten, den möglichen Körperstrom (I_{B}) verursachenden Stromversorgung (12), falls die Gefährdung besteht.

9. Vorrichtung (2) nach Anspruch 8,
**gekennzeichnet durch**
eine oder mehrere der folgenden Sensoreinrichtungen (6): Beschleunigungssensor, Herzfrequenzsensor, Blutdruckmessgerät, Körpertemperatursensor.

10. Vorrichtung (2) nach Anspruch 8 oder 9,
**dadurch gekennzeichnet,**
**dass** die Bewertungseinrichtung (8) konfiguriert ist zur Zusammenführung der Sensordaten (20) der von verschiedenen Sensoreinrichtungen (6) erfassten physikalisch-medizinischen Größen.

11. Vorrichtung (2) nach einem der Ansprüche 8 bis 10,
**dadurch gekennzeichnet,**
**dass** die Bewertungseinrichtung (8) konfiguriert ist zur vorausschauenden Erkennung eines möglichen, oberhalb einer normativ festgelegten Loslass-Schwelle liegenden Körperstroms.

12. Vorrichtung (2) nach einem der Ansprüche 8 bis 11,
**dadurch gekennzeichnet,**
**dass** die Bewertungseinrichtung (8) konfiguriert ist zum Bewerten der Sensordaten (20) mittels digitaler Signalverarbeitungsalgorithmen.

13. Vorrichtung (2) nach einem der Ansprüche 8 bis 12,
**gekennzeichnet durch**
eine Sensordaten-Übertragungseinrichtung (14) zur drahtgebundenen oder drahtlosen Übertragung der Sensordaten (20).

14. Vorrichtung (2) nach einem der Ansprüche 8 bis 13,
**gekennzeichnet durch**
eine Abschaltsignal-Übertragungseinrichtung (16) zur Zuordnung und Abschaltung der Stromversorgung (12) mittels eines drahtgebundenen oder drahtlosen Übertragungsverfahrens.
